(19) Europäisches Patentamt
European Patent Office
Office européen des brevets

(11) Publication number : **0 341 954 B1**

(12) **EUROPEAN PATENT SPECIFICATION**

(45) Date of publication of patent specification : **25.03.92 Bulletin 92/13**

(51) Int. Cl.$^5$ : **A01N 43/653,** A01N 43/50, B27K 3/38, C08K 5/34, C09D 5/14, C14C 9/00, D06M 13/322, D21H 21/36

(21) Application number : **89304629.2**

(22) Date of filing : **08.05.89**

(54) **Biocidal compositions for industrial materials.**

(30) Priority : **10.05.88 JP 111713/88**
**12.08.88 JP 200051/88**

(43) Date of publication of application : **15.11.89 Bulletin 89/46**

(45) Publication of the grant of the patent : **25.03.92 Bulletin 92/13**

(84) Designated Contracting States : **BE CH DE FR GB IT LI**

(56) References cited : **GB-A- 2 180 236**

(73) Proprietor : **KUREHA KAGAKU KOGYO KABUSHIKI KAISHA**
**1-9-11, Nihonbashi, Horidome-cho**
**Chuo-ku Tokyo 103 (JP)**

(72) Inventor : **Arahira, Masato**
**55-1, Sekishita Nishiki-machi**
**Iwaki-shi Fukushima-ken 974 (JP)**
Inventor : **Saishoji, Toshihide**
**154-1, Harada Nishiki-machi**
**Iwaki-shi Fukushima-ken 974 (JP)**
Inventor : **Ohsugi, Katsuhisa**
**3-10-13-404, Nerima**
**Nerima-ku Tokyo 176 (JP)**
Inventor : **Kumazawa, Satoru**
**61-4, Minamidal Kanayama-machi**
**Iwaki-shi Fukushima-ken 974 (JP)**

(74) Representative : **Jones, Helen Marjorie Meredith et al**
**Gill Jennings & Every 53-64 Chancery Lane**
**London WC2A 1HN (GB)**

EP 0 341 954 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to the use of biocidal compositions for preventing deterioration of industrial materials.

2) Description of the Related Art:

It has already been disclosed by the present inventors in GB 2180236 A that the compounds represented by the following formula (A) have plant disease controlling effects, plant growth controlling effects and herbicidal effects and can hence be used as agricultural and horticultural chemicals.

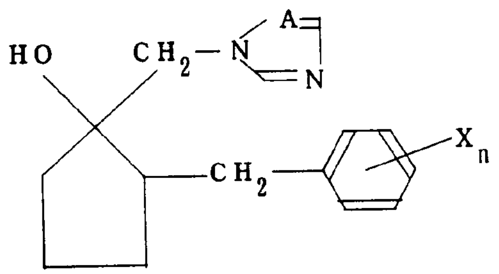

(A)

wherein X means a halogen atom or a $C_1$-$C_5$ alkyl, haloalkyl, phenyl, cyano or nitro group, n stands for 0 or an integer of 1-5, A denotes a nitrogen atom or CH, and when n is an integer of 2-5, Xs may be the same or different.

The present inventors also studied a group of azole derivatives including the compounds represented by the formula (A). As a result, it was found that in addition to the compounds represented by the formula (A), such azole derivatives also include those useful as agricultural and horticultural chemicals and further those not only capable of acting as an agricultural and horticultural chemical but also having effective antifungal activities in animals including human being [Japanese Patent Application Nos. 317754/1987 (corresponding to European Patent Application No. 88305090.8), 6054/1988 and 7822/1988].

By the way, industrial materials, for example, natural materials such as paper, fibers, lumber and leather, synthetic materials such as paints and plastic materials, metal materials, inorganic materials and products formed of such materials are often contaminated by noxious microorganisms and deteriorated in quality. There has hence been a strong demand for the development of chemicals effective against microorganisms noxious to these industrial materials.

The present inventors have proceeded with a further investigation on the utility of the azole derivatives mentioned above. As a result, it has been found that a certain groups of azole derivatives have strong activities against microorganisms noxious to such industrial materials as referred to above, said microorganisms being totally different in field from microorganisms causing disease on agricultural and horticultural crops or fungi causing disease on human being and animals, leading to completion of this invention.

## SUMMARY OF THE INVENTION

An object of this invention is to provide a method for preventing deterioration of industrial materials by using a biocidal composition effective against microorganisms which are noxious to industrial materials.

In one aspect of this invention, there is thus provided the use as a biocidal composition for an industrial material, of an azole derivative represented by the following formula (I):

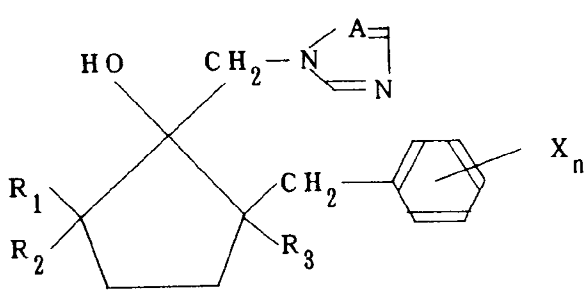

(I)

wherein X means a halogen atom or a $C_1$-$C_5$ alkyl, haloalkyl, phenyl, cyano or nitro group, n stands for 0 or an

integer of 1-5, A denotes a nitrogen atom or CH, $R_1$ and $R_2$ mean individually a hydrogen atom or a $C_1$-$C_5$ alkyl group, $R_3$ denotes a hydrogen atom or a $C_1$-$C_3$ alkyl group, and when n is an integer of 2-5, Xs may be the same or different.

In another aspect of this invention, there is also provided a method for preventing deterioration of an industrial material, which comprises applying to the industrial material a biocidally-effective amount of the azole derivative represented by the formula (I).

In the present invention, the azole derivative represented by the formula (I) has as its structural feature an azolylmethyl group at the 1-position of the cyclopentane ring, a substituted benzyl group or both a substituted benzyl group and a $C_1$-$C_3$ alkyl group at the 2-position, and a hydrogen atom or $C_1$-$C_5$ alkyl group at the 5-position. The above azole derivative useful in the practice of this invention therefore includes stereoisomers such as geometric isomers, i.e., cis-isomer and trans-isomer and optical isomers. This invention embraces not only single use of these isomers but also the use of mixtures containing individual isomers at desired ratios. In other words, it should be noted that the biocidal composition according to this invention contains these isomers either singly or in combination as active ingredient or ingredients. Since the above azole derivative contains a 1,2,4-triazole ring or imidazole ring, the azole derivative can be used in a form such as an acid addition salt with an inorganic acid or organic acid, a metal complex or the like.

As will become apparent from results of tests to be described subsequently, the compound represented by the formula (I) exhibits strong activities against noxious microorganisms which contaminate industrial materials and cause their quality deterioration. When the biocidal composition according to this invention is applied, for example, to a natural material such as paper, fibers, lumber or leather, a synthetic material such as a paint coating, rubber or synthetic resin, a metallic material, inorganic material, or a product formed of the above-mentioned material, it can effectively prevent contamination or quality deterioration of such an industrial material.

## DETAILED DESCRIPTION OF PREFERRED EMBODIMENTS

As illustrative examples of the azole derivative represented by the formula (I) and useful in the practice of this invention, may be mentioned compounds shown below in Table 1, in which the designated types of stereoisomers, namely, A type and B type mean those having the following structures respectively.

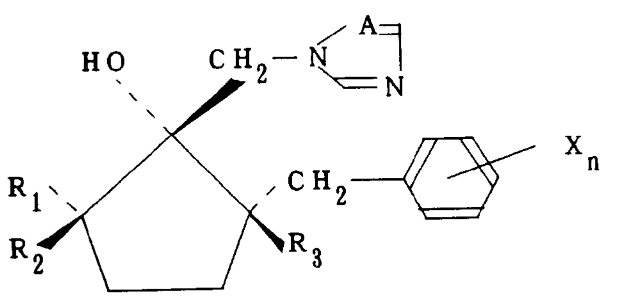

A type

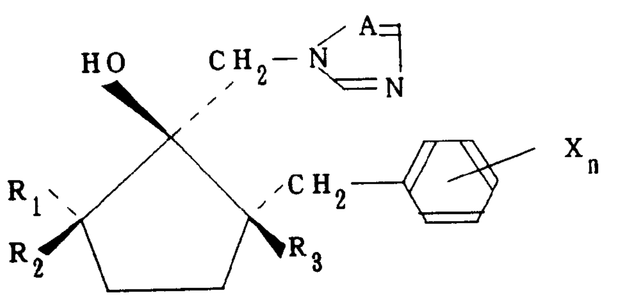

B type

-----: extending below the plane of the page

———: extending on the plane of the page

▬▬: extending above the plane of the page

Table 1

| Compound No. | Variables in formula (I) | | | | | Type of stereoisomer | Melting point (°C) |
|---|---|---|---|---|---|---|---|
| | $R_1$ | $R_2$ | $R_3$ | Xn | A | | |
| 1 | $CH_3$ | $CH_3$ | H | 4-Cℓ | N | A | 113-114 |
| 2 | $CH_3$ | $CH_3$ | H | 4-Cℓ | N | B | 113-114 |
| 3 | $CH_3$ | $CH_3$ | H | 4-Cℓ | CH | A | 133-134 |
| 4 | $CH_3$ | $CH_3$ | H | 4-Cℓ | CH | B | 133-134 |
| 5 | $CH_3$ | $CH_3$ | H | 4-Br | N | A | 129-130 |
| 6 | $CH_3$ | $CH_3$ | H | 4-Br | N | B | 134-135 |
| 7 | $CH_3$ | $CH_3$ | H | 4-Br | CH | A | 149-150 |
| 8 | $CH_3$ | $CH_3$ | H | 4-Br | CH | B | 134-135 |
| 9 | $CH_3$ | $CH_3$ | H | 4-F | N | A | 135-136 |
| 10 | $CH_3$ | $CH_3$ | H | 4-F | N | B | 134-135 |
| 11 | $CH_3$ | $CH_3$ | H | 4-F | CH | A | 131-133 |
| 12 | $CH_3$ | $CH_3$ | H | 4-F | CH | B | 104-106 |
| 13 | $CH_3$ | $CH_3$ | H | 2,4-$Cℓ_2$ | N | A | 126-127 |
| 14 | $CH_3$ | $CH_3$ | H | 2,4-$Cℓ_2$ | N | B | 108-110 |
| 15 | $CH_3$ | $CH_3$ | H | 2,4-$Cℓ_2$ | CH | A | 131-132 |
| 16 | $CH_3$ | H | H | 4-Cℓ | N | A | 100-102 |
| 17 | $CH_3$ | H | H | 4-Cℓ | CH | A | 118-119 |
| 18 | H | $CH_3$ | H | 4-Cℓ | N | A | 75- 76 |
| 19 | H | $CH_3$ | H | 4-Cℓ | N | B | 79- 81 |

Table 1 (cont'd)

| Compound No. | Variables in formula (I) | | | | | Type of stereoisomer | Melting point (°C) |
|---|---|---|---|---|---|---|---|
| | $R_1$ | $R_2$ | $R_3$ | Xn | A | | |
| 20 | $CH_3$ | H | H | 4-Cℓ | N | B | oily substance |
| 21 | $CH_3$ | $CH_3$ | H | H | N | A | oily substance |
| 22 | $CH_3$ | $CH_3$ | H | H | CH | A | 128-130 |
| 23 | $CH_3$ | $CH_3$ | H | 4-$CH_3$ | N | A | 123-124 |
| 24 | $CH_3$ | $CH_3$ | H | 4-$CH_3$ | N | B | 114-115 |
| 25 | $CH_3$ | $CH_3$ | H | 4-$CH_3$ | CH | A | 132-133 |
| 26 | $CH_3$ | $CH_3$ | H | 4-$CH_3$ | CH | B | 130-131 |
| 27 | $CH_3$ | $CH_3$ | H | 2-F, 4-Cℓ | N | A | 129-130 |
| 28 | $CH_3$ | $CH_3$ | H | 2-F, 4-Cℓ | CH | A | 152-154 |
| 29 | $C_2H_5$ | H | H | 4-Cℓ | N | A | 82- 84 |
| 30 | H | $C_2H_5$ | H | 4-Cℓ | N | A | 93- 95 |
| 31 | H | $C_2H_5$ | H | 4-Cℓ | N | B | 76- 78 |
| 32 | $C_2H_5$ | H | H | 4-Cℓ | N | B | 110-112 |
| 33 | $C_2H_5$ | $C_2H_5$ | H | 4-Cℓ | N | A | 124-126 |
| 34 | $C_2H_5$ | $C_2H_5$ | H | 4-Cℓ | N | B | 143-145 |
| 35 | $C_2H_5$ | $C_2H_5$ | H | 4-Cℓ | CH | A | oily substance |
| 36 | $C_2H_5$ | $C_2H_5$ | H | 4-Cℓ | CH | B | 143-145 |
| 37 | n-$C_3H_7$ | H | H | 4-Cℓ | N | A | 83- 85 |

Table 1 (cont'd)

| Compound No. | Variables in formula (I) $R_1$ | $R_2$ | $R_3$ | Xn | A | Type of stereoisomer | Melting point (°C) |
|---|---|---|---|---|---|---|---|
| 38 | H | $n\text{-}C_3H_7$ | H | 4-Cℓ | N | A | 75- 77 |
| 39 | $n\text{-}C_3H_7$ | H | H | 4-Cℓ | CH | A | 115-117 |
| 40 | $C_2H_5$ | H | H | 2,4-$Cℓ_2$ | N | A | 124-127 |
| 41 | $C_2H_5$ | H | H | 2,4-$Cℓ_2$ | CH | A | 111-113 |
| 42 | $C_2H_5$ | H | H | 4-F | N | A | 73- 74 |
| 43 | $C_2H_5$ | H | H | 4-F | CH | A | 111-113 |
| 44 | $C_2H_5$ | H | H | 4-Br | N | A | 80- 82 |
| 45 | $C_2H_5$ | H | H | 4-Br | CH | A | 117-119 |
| 46 | $C_2H_5$ | H | H | 4-$C_6H_5$ | N | A | 107-109 |
| 47 | $C_2H_5$ | H | H | 4-$C_6H_5$ | CH | A | 169-170 |
| 48 | $C_2H_5$ | H | H | 4-t-$C_4H_9$ | N | A | oily substance |
| 49 | $C_2H_5$ | H | H | 4-t-$C_4H_9$ | CH | A | 132-133 |
| 50 | $i\text{-}C_3H_7$ | H | H | 4-Cℓ | N | A | 91- 92 |
| 51 | $n\text{-}C_5H_{11}$ | H | H | 4-Cℓ | N | A | oily substance |
| 52 | $n\text{-}C_5H_{11}$ | H | H | 4-Cℓ | CH | A | 92- 95 |
| 53 | $C_2H_5$ | H | H | 4-Cℓ | CH | B | 138-140 |
| 54 | H | $n\text{-}C_5H_{11}$ | H | 4-Cℓ | N | A | oily substance |
| 55 | $CH_3$ | $CH_3$ | H | 4-$C_6H_5$ | N | A | 122-124 |

Table 1 (cont'd)

| Compound No. | | Variables in formula (I) | | | | | Type of stereoisomer | Melting point (°C) |
|---|---|---|---|---|---|---|---|---|
| | | $R_1$ | $R_2$ | $R_3$ | Xn | A | | |
| 56 | | $CH_3$ | $CH_3$ | H | 4-$C_6H_5$ | N | B | 116-118 |
| 57 | | $CH_3$ | $CH_3$ | H | 4-$C_6H_5$ | CH | A | 162-163 |
| 58 | | $CH_3$ | $CH_3$ | H | 4-$C_6H_5$ | CH | B | 165-167 |
| 59 | | i-$C_3H_7$ | H | H | 4-Cℓ | CH | A | oily substance |
| 60 | | $CH_3$ | $CH_3$ | H | 4-t-$C_4H_9$ | N | A | 107-108 |
| 61 | | $CH_3$ | $CH_3$ | H | 4-t-$C_4H_9$ | CH | A | 167-168 |
| 62 | | H | i-$C_3H_7$ | H | 4-Cℓ | N | B | oily substance |
| 63 | | H | i-$C_3H_7$ | H | 4-Cℓ | N | A | 102-103 |
| 64 | | H | i-$C_3H_7$ | H | 4-Cℓ | CH | A | 146-147 |
| 65 | | i-$C_3H_7$ | H | H | 4-Cℓ | N | B | 120-121 |
| 66 | | n-$C_4H_9$ | H | H | 4-Cℓ | CH | A | oily substance |
| 67 | | H | n-$C_4H_9$ | H | 4-Cℓ | N | A | 94- 95 |
| 68 | | H | n-$C_4H_9$ | H | 4-Cℓ | N | B | oily substance |
| 69 | | i-$C_4H_9$ | H | H | 4-Cℓ | N | A | oily substance |
| 70 | | i-$C_4H_9$ | H | H | 4-Cℓ | CH | A | oily substance |
| 71 | | n-$C_4H_9$ | H | H | 4-Cℓ | N | A | oily substance |
| 72 | Isomer a | $CH_3$ | $C_2H_5$ | H | 4-Cℓ | N | A | 72-a/72-b mixture 98-101 |
| | Isomer b | $C_2H_5$ | $CH_3$ | H | 4-Cℓ | N | A | |

Table 1 (cont'd)

| Compound No. | | Variables in formula (I) | | | | | Type of stereoisomer | Melting point (°C) |
|---|---|---|---|---|---|---|---|---|
| | | $R_1$ | $R_2$ | $R_3$ | Xn | A | | |
| 73 | Isomer a | $CH_3$ | $C_2H_5$ | H | 4-Cℓ | N | B | 73-a/73-b mixture 117-119 |
| | Isomer b | $C_2H_5$ | $CH_3$ | H | 4-Cℓ | N | B | |
| 74 | Isomer a | $CH_3$ | $C_2H_5$ | H | 4-Cℓ | CH | B | 74-a/74-b mixture 122-127 |
| | Isomer b | $C_2H_5$ | $CH_3$ | H | 4-Cℓ | CH | B | |
| 75 | | $CH_3$ | $CH_3$ | H | 4-t-$C_4H_9$ | N | B | oily substance |
| 76 | | $CH_3$ | $CH_3$ | H | 4-t-$C_4H_9$ | CH | B | 132-133 |
| 77 | | H | H | H | H | N | A | 140-141 |
| 78 | | H | H | H | H | CH | A | 130-131 |
| 79 | | H | H | H | 4-$CH_3$ | N | A | 128-129 |
| 80 | | H | H | H | 4-$CH_3$ | CH | A | 122-123 |
| 81 | | H | H | H | 4-t-$C_4H_9$ | N | A | 129-130 |
| 82 | | H | H | H | 4-t-$C_4H_9$ | CH | A | 123-124 |
| 83 | | H | H | H | 2-Cℓ | N | A | 154-155 |
| 84 | | H | H | H | 2-Cℓ | CH | A | 103-104 |
| 85 | | H | H | H | 3-Cℓ | N | A | 152-153 |
| 86 | | H | H | H | 3-Cℓ | CH | A | 105-106 |

Table 1 (cont'd)

| Compound No. | Variables in formula (I) | | | | | Type of stereoisomer | Melting point (°C) |
|---|---|---|---|---|---|---|---|
| | $R_1$ | $R_2$ | $R_3$ | Xn | A | | |
| 87 | H | H | H | 4-Cℓ | N | A | 115-116 |
| 88 | H | H | H | 4-Cℓ | CH | A | 115-116 |
| 89 | H | H | H | 2,4-$Cℓ_2$ | N | A | 120-121 |
| 90 | H | H | H | 2,4-$Cℓ_2$ | CH | A | 150-151 |
| 91 | H | H | H | 4-F | N | A | 135-136 |
| 92 | H | H | H | 4-F | CH | A | 139-140 |
| 93 | H | H | H | 2,4-$F_2$ | N | A | 118-119 |
| 94 | H | H | H | 2,4-$F_2$ | CH | A | 144-145 |
| 95 | H | H | H | 2,6-$F_2$ | N | A | 104-105 |
| 96 | H | H | H | 2,6-$F_2$ | CH | A | 150-151 |
| 97 | H | H | H | 3,4-$F_2$ | N | A | 119-121 |
| 98 | H | H | H | 3,4-$F_2$ | CH | A | 103-105 |
| 99 | H | H | H | 2,3,4,5,6-$F_5$ | N | A | 118-120 |
| 100 | H | H | H | 3-$CF_3$ | N | A | 152-153 |
| 101 | H | H | H | 3-$CF_3$ | CH | A | 87- 88 |
| 102 | H | H | H | 2-F, 4-Cℓ | N | A | 125-127 |
| 103 | H | H | H | 2-F, 4-Cℓ | CH | A | 141-143 |
| 104 | H | H | H | 4-Br | N | A | 106-107 |

Table 1 (cont'd)

| Compound No. | Variables in formula (I) R1 | R2 | R3 | Xn | A | Type of stereoisomer | Melting point (°C) |
|---|---|---|---|---|---|---|---|
| 105 | H | H | H | 4-Br | CH | A | 119-120 |
| 106 | H | H | H | 4-$C_6H_5$ | N | A | 146-147 |
| 107 | H | H | H | 4-$C_6H_5$ | CH | A | 182-183 |
| 108 | H | H | H | 4-$NO_2$ | N | A | 131-132 |
| 109 | H | H | H | 4-CN | N | A | 115-116 |
| 110 | H | H | H | 4-CN | CH | A | 103-104 |
| 111 | $CH_3$ | $CH_3$ | $CH_3$ | 4-Cℓ | N | A | 159-160 |
| 112 | $CH_3$ | $CH_3$ | $CH_3$ | 4-Cℓ | N | B | 178-179 |
| 113 | $CH_3$ | $CH_3$ | $CH_3$ | 4-Cℓ | CH | A | 186-187 |
| 114 | $CH_3$ | $CH_3$ | $CH_3$ | 4-Cℓ | CH | B | 157-158 |

The azole derivatives useful in the practice of this invention can each be prepared by reacting an oxirane compound, which is represented by the following formula (II):

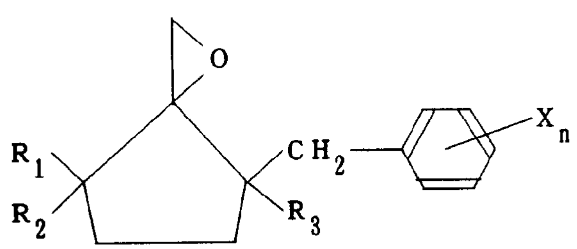

(II)

wherein X, $R_1$, $R_2$, $R_3$ and n have the same meanings as defined above, with a compound represented by the following formula (III):

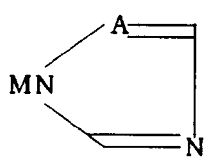

(III)

wherein M means a hydrogen atom or alkali metal and A has the same meaning as defined above, in the presence of a diluent.

Incidentally, the oxirane compound represented by the formula (II) can be obtained by reacting a cyclopentanone represented by formula (IV) below, for example, with dimethyloxosulfonium methylide or dimethylsulfonium methylide in the presence of a diluent, for example, in light of the process described in Organic Synthesis, 49, 78 (1968) and Journal of American Chemical Society, 1353 (1965).

Still more, as a different method, there is a method by which a methylenecyclopentane represented by the formula (V) is obtained from a cyclopentanone represented by the formula (IV) through the Witting reaction [refer to Org. Syn. 40, 66 (1966) and J. Org. Chem. 28, 1128 (1963)], and then the oxirane derivative represented by the formula (II) can be obtained from the thus prepared compound by the epoxidation [refer to Org. Syn. Coll. Vol., 4, 552 (1963) and Org. Syn., 49, 62 (1969)].

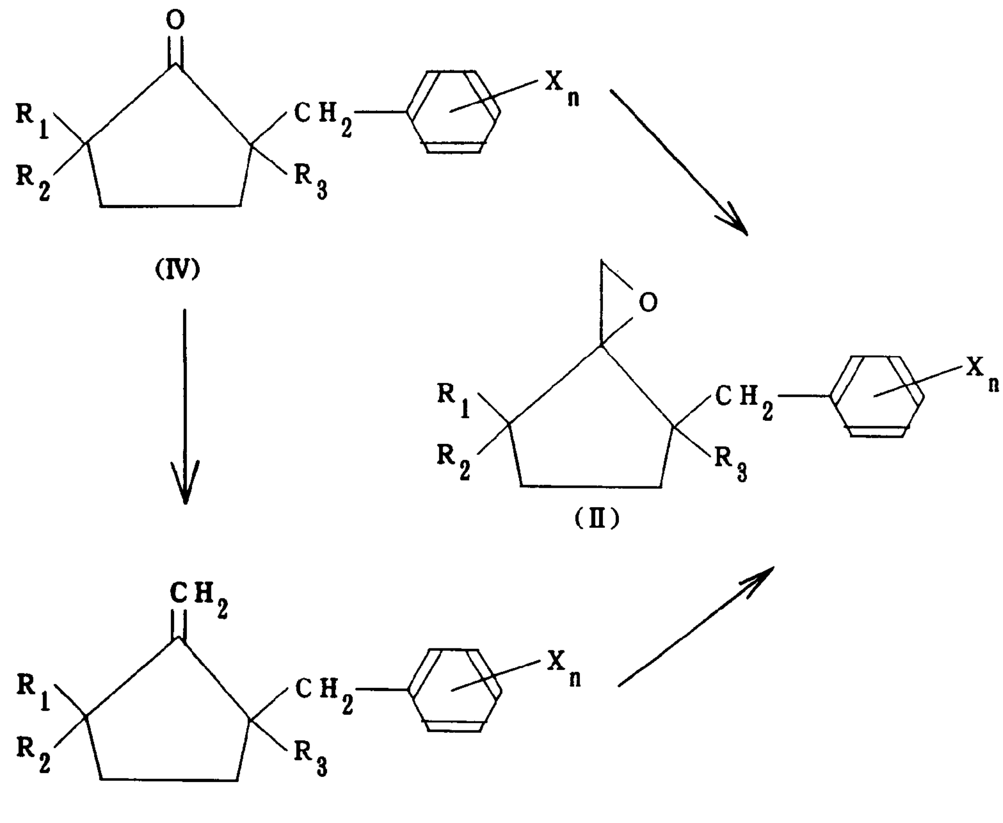

(IV)

(II)

(V)

wherein X, $R_1$, $R_2$, $R_3$ and n have the same meanings as defined above.

The biocidal compositions for industrial materials, said compositions pertaining to the present invention, show controlling effects against microorganisms which grow on the industrial materials and cause deterioration of their base materials. Such microorganisms may include, for example, paper/pulp-deteriorating microorganisms including slime-forming microorganisms, such as Aspergillus sp., Trichoderma sp., Penicillium sp., Geotrichum sp., Chaetomium sp., Cadophora sp., Ceratostomella sp., Cladosporium sp., Corticium sp., Lentinus sp., Lezites sp., Phoma sp., Polysticus sp., Pullularia sp., Stereum sp., Trichosporium sp., Aerobacter sp., Bacillus sp., Desulfovibrio sp., Pseudomonas sp., Flavobacterium sp. and Micrococcus sp.; fibers-deteriorating microorganisms such as Aspergillus sp., Penicillium sp., Chaetomium sp., Myrothecium sp., Curvularia sp., Gliomastix sp., Memnoniella sp., Sarcopodium sp., Stachybotrys sp., Stemphylium sp., Zygorhynchus sp., Bacillus sp. and Staphylococcus sp.; lumber-deteriorating microorganisms such as Tyromyces palustris, Coriolus versicolor, Aspergillus sp., Penicillium sp., Rhizopus sp., Aureobasidium sp., Gliocladium sp., Cladosporium sp., Chaetomium sp. and Trichoderma sp.; leather-deteriorating microorganisms such as Aspergillus sp., Penicillium sp., Chaetomium sp., Cladosporium sp., Mucor sp., Paecilomyces sp., Pilobus sp., Pullularia sp., Trichosporon sp. and Tricothecium sp.; rubber/plastics-deteriorating microorganisms such as Aspergillus sp., Penicillium sp., Rhizopus sp., Trichoderma sp., Chaetomium sp., Myrothecium sp., Streptomyces sp., Pseudomonus sp., Bacillus sp., Micrococcus sp., Serratia sp., Margarinomyces sp. and Monascus sp.; paint-deteriorating microorganisms such as Aspergillus sp., Penicillium sp., Cladosporium sp., Aureobasidium sp., Gliocladium sp., Botryodiplodia sp., Macrosporium sp., Monilia sp., Phoma sp., Pullularia sp., Sporotrichum sp., Trichoderma sp., Bacillus sp., Proteus sp., Pseudomonas sp. and

Serratia sp.

The biocidal compositions for industrial materials, said compositions pertaining to the present invention, can each be formulated by dissolving or dispersing an effective amount of the compound represented by the formula (I) in a suitable liquid vehicle or mixing it with a solid vehicle and optionally adding an emulsifier, dispersant, spreading agent, penetrant, wetting agent, stabilizer and/or the like. One or more of other biocides, insecticides, deterioration inhibitors and the like may also be incorporated.

Any liquid may be used as a liquid vehicle so long as it does not react with the effective ingredient. It is possible to use, for example, water, an alcohol such as methyl alcohol, ethyl alcohol, ethylene glycol or cellosolve, a ketone such as acetone or methyl ethyl ketone, an ether such as dimethyl ether, diethyl ether, dioxane or tetrahydrofuran, an aromatic hydrocarbon such as benzene, toluene, xylene or methylnaphthalene, an aliphatic hydrocarbon such as gasoline, kerosine, lamp oil, stove oil, furnace oil, machine oil or fuel oil, an acid amide such as dimethylformamide or N-methylpyrrolidone, a halogenated hydrocarbon such as chloroform or carbon tetrachloride, an ester such as ethyl acetate or the glycerin ester of a fatty acid, a nitrile such as acetonitrile, dimethylsulfoxide, or the like. As a solid vehicle on the other hand, it is possible to use fine particulate or granular material such as clay, kaolin clay, bentonite, acid clay, pyrophyllite, talc, diatomaceous earth, calcite, urea or ammonium sulfate. As an emulsifier or dispersant, it is possible to use a surfactant such as a soap, alkyl sulfonate, alkyl arylsulfonate, dialkyl sulfosuccinate, quaternary ammonium salt, oxyalkylamine, fatty acid ester, polyalkylene-oxide-type surfactant or anhydrosorbitol-type surfactant.

Upon formulation of the azole derivative of the formula (I) as an effective ingredient into a biocidal composition according to this invention, it is generally suitable to add it to give a concentration of 0.1-99.9 wt.% although its content may vary depending on the preparation form and application purpose of the biocidal composition.

In the present invention, a biocidally-effective amount of the azole derivative of the formula (I) can be applied in a preparation form, for example, as a wettable powder, dust, granules, paste, suspension or spray to an industrial material. The application to the industrial material can be effected, for example, by spraying or coating the surface of paper, pulp, fibers, lumber, leather, rubber product, synthetic resin product or coating. The biocidally-effective amount may be generally 0.005-5 wt.% or preferably 0.01-1 wt.%, both, based on an industrial material to be treated.

## Examples

### Preparation Example:

Preparation of a typical azole derivative of the formula (I) will hereinafter be described. It should however be noted that the other azole derivatives of the formula (I) can also be prepared likewise.

### Preparation of 2-(4-chlorobenzyl)-2,5,5-trimethyl-1-(1H-1,2,4-triazol-1-ylmethyl)-1-cyclopentanol (Compound No. 111 in Table 1)

Anhydrous dimethylformamide (3 mℓ) was added with 230 mg of sodium hydride and further with 390 mg of 1H-1,2,4-triazole. The resultant mixture was stirred at room temperature until foaming subsided. The resulting solution was added with 2 mℓ of an anhydrous dimethylformamide solution which contained 1.0 g of 4-(4-chlorobenzyl)-4,7,7-trimethyl-1-oxaspiro[2.4]heptane, followed by stirring at 120°C for 24 hours. After allowing the thus-obtained reaction mixture to cool down, it was poured into ice water and then extracted with methylene chloride to obtain an organic layer. After washing the organic layer with aqueous saline solution, it was dried over anhydrous sodium sulfate, and the solvent was thereafter distilled off under reduced pressure. The thus-obtained residue was subjected to and purified by chromatography on a silica gel column (eluent: ethyl acetate). It was then recrystallized from n-hexane-ethyl acetate so that 1.06 g of the target compound was obtained.

By the way, the raw material,4-(4-chlorobenzyl)-4,7,7-trimethyl-1-oxaspiro[2.4]heptane, was obtained in the following manner.

2-(4-Chlorobenzyl)-2,5,5-trimethyl-1-methylene-cyclopentane (6.1 g) was dissolved in 60 mℓ of chloroform, followed by the addition of 8.5 g of m-chloroperbenzoic acid. The resultant mixture was stirred for 5 hours under ice cooling. Calcium hydroxide (4.0 g) was added, followed by stirring at room temperature for 30 minutes. A solid thus precipitated was filtered off. From the chloroform layer as the filtrate, chloroform was distilled off under reduced pressure to obtain a colorless oily substance. The substance was subjected to and purified by chromatography on a silica gel column (eluent: 6:1 mixed solvent of n-hexane and methylene chloride), thereby obtaining 2.0 g of 4-(4-chlorobenzyl)-4,7,7-trimethyl-1-oxaspiro[2.4]heptane used in the above Preparation Example.

Biocidal Compositions and Tests:

The present invention will hereinafter be described further by the following exemplary biocidal compositions and tests.

Composition 1:

A dust was obtained by grinding and mixing 1 wt.% of an invention compound (Compound No. 87), 89 wt.% of kaolin clay and 10 wt.% of talc.

Composition 2:

A wettable powder was obtained by grinding and mixing 25 wt.% of an invention compound (Compound No. 1), 2 wt.% of sodium ligninsulfonate, 2 wt.% of sodium lauryl sulfate and 71 wt.% of clay.

Test 1:

Each test compound according to this invention was added to a modified Mueller-Hinton culture medium (product of Nissui Seiyaku Kabushiki Kaisha) to give a predetermined concentration. The resultant culture medium was poured into Petri dishes and was caused to coagulate. They were inoculated with three kinds of noxious microorganisms (Staphvlococcus sp., Bacillus sp. and Micrococcus sp.) respectively, followed by incubation at 37°C. Depending on whether those microorganisms had grown or not on the following day, the minimum inhibitory concentrations of the invention compound for the respective noxious microorganisms were determined. Based on the minimum inhibitory concentrations thus obtained, 5-stage ranking was conducted in accordance with the following system:

Minimum inhibitory concentration

1 .... at least 100 ppm
2 .... at least 50 ppm but lower than 100 ppm
3 .... at least 12.5 ppm but lower than 50 ppm
4 .... at least 3.13 ppm but lower than 12.5 ppm
5 .... lower than 3.13 ppm

Table 2

| Sample compound (No. in Table 1) | Test microorganism | | |
|---|---|---|---|
| | S.sp. | B.sp. | M.sp. |
| 1 | 2 | 3 | 3 |
| 3 | 2 | 3 | 5 |
| 4 | 2 | 3 | 5 |
| 5 | 2 | 3 | 4 |
| 6 | 2 | 2 | 3 |
| 7 | 3 | 3 | 4 |
| 8 | 2 | 2 | 4 |
| 9 | 1 | 2 | 3 |
| 10 | 1 | 2 | 2 |
| 11 | 1 | 3 | 5 |
| 12 | 1 | 3 | 4 |
| 13 | 3 | 3 | 4 |
| 14 | 3 | 3 | 4 |
| 16 | 2 | 2 | 3 |
| 17 | 2 | 3 | 4 |
| 19 | 2 | 1 | 2 |
| 23 | 1 | 2 | 3 |
| 24 | 2 | 2 | 3 |
| 25 | 2 | 3 | 4 |
| 26 | 2 | 3 | 4 |
| 27 | 2 | 3 | 3 |
| 28 | 3 | 4 | 5 |
| 29 | 2 | 2 | 3 |
| 30 | 2 | 2 | 4 |
| 31 | 2 | 2 | 2 |
| 32 | 3 | 2 | 3 |
| 34 | 3 | 3 | 4 |
| 36 | 4 | 5 | 5 |
| 37 | 3 | 3 | 4 |
| 38 | 3 | 3 | 4 |
| 39 | 3 | 4 | 5 |
| 40 | 3 | 3 | 4 |
| 41 | 4 | 5 | 5 |
| 42 | 1 | 2 | 2 |
| 43 | 1 | 3 | 4 |
| 44 | 3 | 3 | 3 |
| 45 | 3 | 4 | 5 |

Table 2 (cont'd)

| Sample compound (No. in Table 1) | Test microorganism | | |
|---|---|---|---|
| | S. sp. | B. sp. | M. sp. |
| 47 | 4 | 5 | 5 |
| 48 | 3 | 3 | 4 |
| 49 | 4 | 4 | 5 |
| 50 | 3 | 3 | 4 |
| 51 | 4 | 3 | 4 |
| 52 | 4 | 4 | 5 |
| 53 | 2 | 1 | 2 |
| 54 | 4 | 4 | 4 |
| 55 | 3 | 3 | 4 |
| 56 | 3 | 3 | 4 |
| 57 | 3 | 4 | 5 |
| 58 | 4 | 4 | 5 |
| 59 | 3 | 4 | 5 |
| 60 | 3 | 4 | 4 |
| 61 | 3 | 4 | 5 |
| 62 | 3 | 2 | 3 |
| 63 | 3 | 2 | 4 |
| 64 | 3 | 3 | 5 |
| 65 | 3 | 3 | 4 |
| 66 | 4 | 3 | 5 |

| Saple compound (No. in Table 1) | Test microorganism | | |
|---|---|---|---|
| | S. sp. | B. sp. | M. sp. |
| 67 | 3 | 3 | 5 |
| 68 | 3 | 2 | 4 |
| 69 | 3 | 3 | 4 |
| 70 | 3 | 3 | 5 |
| 71 | 4 | 3 | 4 |
| 75 | 3 | 3 | 4 |
| 76 | 3 | 4 | 4 |
| 78 | 1 | 1 | 3 |
| 80 | 1 | 2 | 3 |
| 81 | 1 | 3 | 3 |
| 82 | 3 | 3 | 5 |
| 84 | 1 | 2 | 3 |
| 86 | 1 | 3 | 4 |
| 90 | 3 | 4 | 5 |
| 92 | 1 | 2 | 3 |
| 94 | 1 | 2 | 3 |

Table 2 (cont'd)

| Sample compound (No. in Table 1) | Test microorganism | | |
|---|---|---|---|
| | S.sp. | B.sp. | M.sp. |
| 96 | 1 | 2 | 3 |
| 98 | 1 | 2 | 4 |
| 101 | 1 | 3 | 4 |
| 103 | 2 | 3 | 4 |
| 104 | 1 | 1 | 3 |
| 105 | 2 | 3 | 4 |
| 107 | 1 | 3 | 5 |

The abbreviation in Table 2 shows each of the following microorganisms:

S.sp.; Staphylococcus sp.
B.sp.; Bacillus sp.
M.sp.; Micrococcus sp.

Test 2:

Each test compound according to this invention was added to a potato-sucrose-agar culture medium to give 100 ppm. The resultant culture medium was poured into Petri dishes and was caused to coagulate. They were inoculated with seven kinds of noxious microorganisms (Aspergillus niger, Aspergillus terreus, Aureobasidium pullulans, Rhizopus stolonifer, Penicillium citrinum, Penicillium funiculosum and Chaetomium globosum) respectively, followed by incubation at 28°C for 7 days. Hyphae elongation inhibitory rates (%) were determined respectively in accordance with the below-described equation. Based on the hyphae elongation inhibitory rates, the test results were ranked in four stages by the following ranking system. The results are shown in Table 3.

$$\text{Hyphae elongation inhibitory rate, \%} = \frac{\text{Hyphae elongation in non-treated group, mm} - \text{Hyphae elongation in treated group group, mm}}{\text{Hyphae elongation in non-treated group, mm}} \times 100$$

Ranking system:

Hyphae elongation inhibitory rate, %

1 .... at least 0% but smaller than 25%
2 .... at least 25% but smaller than 50%
3 .... at least 50% but smaller than 75%
4 .... at least 75% and up to 100%

Table 3

| Sample compound (No. in Table 1) | Test microorganism | | | | | | |
|---|---|---|---|---|---|---|---|
| | Asp.t. | Asp.n. | Aur.p. | Rhi.s. | Pen.c. | Pen.f. | Chae.g. |
| 1 | 4 | 4 | 4 | 4 | 4 | 4 | 4 |
| 2 | 4 | 4 | 4 | 4 | 2 | 4 | 4 |
| 3 | 4 | 4 | 4 | 4 | 4 | 4 | 4 |
| 4 | 4 | 4 | 4 | 4 | 4 | 4 | 4 |
| 5 | 4 | 4 | 4 | 4 | 4 | 4 | 4 |
| 6 | 4 | 4 | 4 | 4 | 2 | 4 | 4 |
| 7 | 4 | 4 | 4 | 4 | 4 | 4 | 4 |
| 8 | 4 | 4 | 4 | 4 | 4 | 4 | 4 |
| 9 | 4 | 4 | 4 | 4 | 4 | 4 | 4 |
| 10 | 4 | 4 | 4 | 4 | 1 | 4 | 4 |
| 11 | 4 | 4 | 4 | 4 | 4 | 4 | 4 |
| 12 | 4 | 4 | 4 | 4 | 4 | 4 | 4 |
| 13 | 4 | 4 | 4 | 4 | 2 | 4 | 4 |
| 14 | 2 | 4 | 4 | 4 | 2 | 4 | 4 |
| 19 | 4 | 4 | 4 | 4 | 2 | 4 | 4 |
| 20 | 4 | 4 | 4 | 4 | 4 | 4 | 4 |
| 21 | 4 | 4 | 4 | 4 | 4 | 4 | 4 |
| 22 | 4 | 4 | 4 | 4 | 4 | 4 | 4 |
| 23 | 4 | 4 | 4 | 4 | 4 | 4 | 4 |

Table 3 (Cont'd)

| Sample compound (No. in Table 1) | Test microorganism | | | | | | |
|---|---|---|---|---|---|---|---|
| | Asp.t. | Asp.n. | Aur.p. | Rhi.s. | Pen.c. | Pen.f. | Chae.g. |
| 24 | 4 | 4 | 4 | 4 | 2 | 4 | 4 |
| 25 | 4 | 4 | 4 | 4 | 4 | 4 | 4 |
| 26 | 4 | 4 | 4 | 4 | 4 | 4 | 4 |
| 27 | 4 | 4 | 4 | 4 | 4 | 4 | 4 |
| 28 | 4 | 4 | 4 | 4 | 4 | 4 | 4 |
| 29 | 4 | 4 | 4 | 4 | 4 | 4 | 4 |
| 30 | 4 | 4 | 4 | 4 | 4 | 4 | 4 |
| 31 | 4 | 4 | 4 | 4 | 4 | 4 | 4 |
| 32 | 4 | 4 | 4 | 4 | 4 | 4 | 4 |
| 33 | 4 | 4 | 4 | 4 | 4 | 4 | 4 |
| 34 | 4 | 4 | 4 | 4 | 4 | 4 | 4 |
| 35 | 4 | 4 | 4 | 4 | 4 | 4 | 4 |
| 36 | 4 | 4 | 4 | 4 | 4 | 4 | 4 |
| 37 | 4 | 4 | 4 | 4 | 4 | 4 | 4 |
| 38 | 4 | 4 | 4 | 4 | 4 | 4 | 4 |
| 39 | 4 | 4 | 4 | 4 | 4 | 4 | 4 |
| 40 | 1 | 4 | 4 | 4 | 4 | 4 | 4 |
| 41 | 4 | 4 | 4 | 4 | 4 | 4 | 4 |
| 42 | 4 | 4 | 4 | 4 | 4 | 4 | 4 |

Table 3 (Cont'd)

| Sample compound (No. in Table 1) | Test microorganism | | | | | | |
|---|---|---|---|---|---|---|---|
| | Asp.t. | Asp.n. | Aur.p. | Rhi.s. | Pen.c. | Pen.f. | Chae.g. |
| 43 | 4 | 4 | 4 | 4 | 4 | 4 | 4 |
| 44 | 4 | 4 | 4 | 4 | 4 | 4 | 4 |
| 45 | 4 | 4 | 4 | 4 | 4 | 4 | 4 |
| 46 | 2 | 4 | 1 | 1 | 4 | 1 | 4 |
| 47 | 4 | 4 | 1 | 1 | 4 | 4 | 4 |
| 48 | 1 | 4 | 4 | 3 | 2 | 1 | 4 |
| 49 | 4 | 4 | 4 | 4 | 4 | 4 | 4 |
| 50 | 4 | 4 | 4 | 4 | 4 | 4 | 4 |
| 51 | 4 | 4 | 4 | 4 | 4 | 4 | 4 |
| 52 | 4 | 4 | 4 | 4 | 4 | 4 | 4 |
| 53 | 4 | 4 | 4 | 4 | 4 | 4 | 4 |
| 54 | 4 | 4 | 4 | 4 | 4 | 3 | 4 |
| 55 | 4 | 4 | 4 | 3 | 4 | 1 | 4 |
| 56 | 2 | 3 | 4 | 3 | 2 | 2 | 4 |
| 57 | 4 | 4 | 1 | 1 | 4 | 4 | 4 |
| 58 | 4 | 4 | 4 | 4 | 4 | 4 | 4 |
| 59 | 4 | 4 | 4 | 4 | 4 | 4 | 4 |
| 60 | 2 | 3 | 3 | 1 | 2 | 1 | 4 |
| 61 | 4 | 4 | 4 | 1 | 4 | 2 | 4 |

Table 3 (Cont'd)

| Sample compound (No. in Table 1) | Test microorganism | | | | | | |
|---|---|---|---|---|---|---|---|
| | Asp.t. | Asp.n. | Aur.p. | Rhi.s. | Pen.c. | Pen.f. | Chae.g. |
| 63 | 4 | 4 | 4 | 4 | 4 | 4 | 4 |
| 64 | 4 | 4 | 4 | 4 | 4 | 4 | 4 |
| 66 | 4 | 4 | 4 | 4 | 4 | 4 | 4 |
| 67 | 4 | 4 | 4 | 4 | 4 | 4 | 4 |
| 68 | 2 | 3 | 4 | 3 | 2 | 1 | 4 |
| 69 | 4 | 4 | 4 | 4 | 4 | 3 | 4 |
| 70 | 4 | 4 | 4 | 4 | 4 | 4 | 4 |
| 71 | 4 | 4 | 4 | 4 | 4 | 4 | 4 |
| 75 | 1 | 2 | 3 | 1 | 1 | 2 | 4 |
| 76 | 4 | 4 | 4 | 4 | 4 | 4 | 4 |
| 77 | 2 | 4 | 4 | 1 | 1 | 2 | 4 |
| 78 | 4 | 4 | 1 | 1 | 3 | 4 | 4 |
| 79 | 4 | 4 | 4 | 1 | 3 | 3 | 4 |
| 80 | 4 | 4 | 4 | 4 | 4 | 3 | 4 |
| 81 | 1 | 3 | 4 | 1 | 3 | 1 | 4 |
| 82 | 1 | 4 | 1 | 1 | 4 | 1 | 4 |
| 84 | 1 | 4 | 4 | 1 | 2 | 3 | 4 |
| 85 | 4 | 4 | 1 | 1 | 4 | 2 | 4 |
| 86 | 4 | 4 | 2 | 1 | 4 | 4 | 4 |

Table 3 (Cont'd)

| Sample compound (No. in Table 1) | Test microorganism | | | | | | |
|---|---|---|---|---|---|---|---|
| | Asp.t. | Asp.n. | Aur.p. | Rhi.s. | Pen.c. | Pen.f. | Chae.g. |
| 87 | 4 | 4 | 4 | 4 | 4 | 4 | 4 |
| 88 | 4 | 4 | 4 | 4 | 4 | 4 | 4 |
| 89 | 1 | 4 | 4 | 4 | 2 | 4 | 4 |
| 90 | 4 | 4 | 4 | 4 | 4 | 4 | 4 |
| 91 | 2 | 4 | 4 | 4 | 1 | 2 | 4 |
| 92 | 4 | 4 | 1 | 4 | 4 | 4 | 4 |
| 94 | 1 | 4 | 1 | 1 | 2 | 3 | 4 |
| 96 | 3 | 4 | 4 | 1 | 2 | 2 | 4 |
| 97 | 4 | 4 | 4 | 4 | 3 | 3 | 4 |
| 98 | 4 | 4 | 1 | 1 | 4 | 4 | 4 |
| 99 | 1 | 4 | 4 | 4 | 1 | 1 | 4 |
| 101 | 4 | 4 | 4 | 1 | 4 | 2 | 4 |
| 102 | 2 | 4 | 4 | 4 | 2 | 4 | 4 |
| 103 | 4 | 4 | 4 | 4 | 4 | 4 | 4 |
| 104 | 4 | 4 | 4 | 4 | 4 | 4 | 4 |
| 105 | 4 | 4 | 4 | 4 | 4 | 4 | 4 |
| 106 | 1 | 4 | 1 | 1 | 4 | 2 | 4 |
| 107 | 4 | 4 | 4 | 1 | 4 | 4 | 4 |
| 109 | 2 | 4 | 4 | 1 | 1 | 1 | 4 |

Table 3 (Cont'd)

| Sample compound (No. in Table 1) | Test microorganism | | | | | | |
|---|---|---|---|---|---|---|---|
| | Asp.t. | Asp.n. | Aur.p. | Rhi.s. | Pen.c. | Pen.f. | Chae.g. |
| 111 | 4 | 4 | 4 | 4 | 4 | 4 | 4 |
| 112 | 1 | 4 | 4 | 1 | 1 | 1 | 4 |
| 113 | 4 | 4 | 4 | 4 | 4 | 4 | 4 |
| 114 | 4 | 4 | 4 | 4 | 4 | 4 | 4 |

The abbreviation in Table 3 shows each of the following microorganisms:

Asp.t.; *Aspergillus terreus*
Asp.n.; *Aspergillus niger*
Aur.p.; *Aureobasidium pullulans*
Rhi.s.; *Rhizopus stolonifer*
Pen.c.; *Penicillium citrinum*
Pen.f.; *Penicillium funiculosum*
Chae.g.; *Chaetomium globosum*

## Claims

1. Use of an azole derivative represented by the following formula (I):

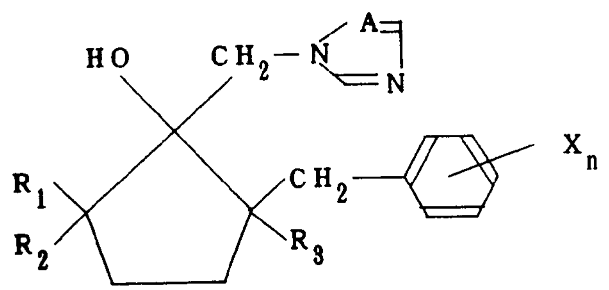

(I)

wherein X means a halogen atom or a $C_1$-$C_5$ alkyl, haloalkyl, phenyl, cyano or nitro group, n stands for 0 or an integer of 1-5, A denotes a nitrogen atom or CH, $R_1$ and $R_2$ mean individually a hydrogen atom or a $C_1$-$C_5$ alkyl group, $R_3$ denotes a hydrogen atom or a $C_1$-$C_3$ alkyl group, and when n is an integer of 2-5, Xs may be the same or different, as a biocide in an industrial material.

2. Use as claimed in Claim 1, wherein the industrial material is selected from paper, pulp, fibers, lumber, leather, rubber, synthetic resins and paints.

3. A method for preventing deterioration of an industrial material, which comprises applying to the industrial material a biocidally-effective amount of an azole derivative represented by the following formula (I):

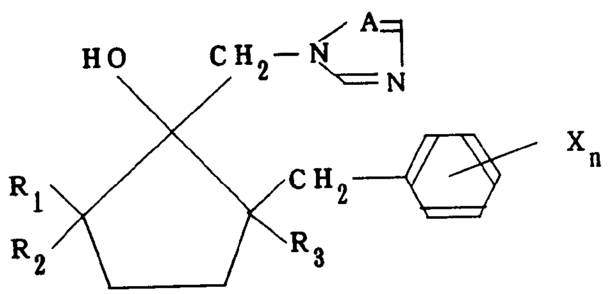

(I)

wherein X means a halogen atom or a $C_1$-$C_5$ alkyl, haloalkyl, phenyl, cyano or nitro group, n stands for 0 or an integer of 1-5, A denotes a nitrogen atom or CH, $R_1$ and $R_2$ mean individually a hydrogen atom or a $C_1$-$C_5$ alkyl group, $R_3$ denotes a hydrogen atom or a $C_1$-$C_3$ alkyl group, and when n is an integer of 2-5, Xs may be the same or different.

4. The method as claimed in Claim 3, wherein the industrial material is selected from paper, pulp, fibers, lumber, leather, rubber, synthetic resins and paints.

## Patentansprüche

1. Verwendung eines Azolderivats der folgenden Formel (I)

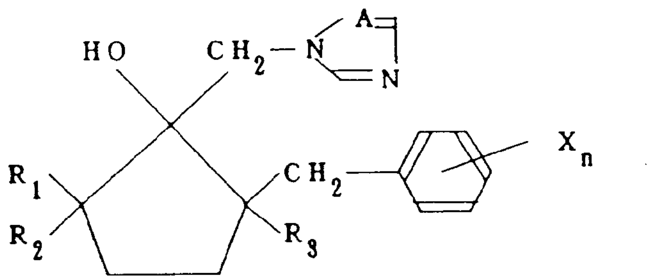

(I)

worin X ein Halogenatom oder eine $C_1$-$C_5$-Alkyl-, Halogenalkyl-, Phenyl-, Cyano- oder Nitrogruppe bedeutet, n für 0 oder eine ganze Zahl von 1 bis 5 steht, A ein Stickstoffatom oder CH darstellt, $R_1$ und $R_2$ individuell ein Wasserstoffatom oder eine $C_1$-$C_5$-Alkylgruppe bedeuten, $R_3$ ein Wasserstoffatom oder eine $C_1$-$C_3$-Alkylgruppe ist und, wenn n eine ganze Zahl von 2 bis 5 ist, die Reste X gleich oder unterschiedlich sein können; als Biozid in einem industriellen Material.

2. Verwendung nach Anspruch 1, worin das industrielle Material ausgewählt ist unter Papier, Zellstoff, Fasern, Holz, Leder, Gummi, kunstharzen und Lacken.

3. Methode zum Verhindern der Schädigung eines industriellen Materials, bei dem auf das industrielle Material eine biozid-wirksame Menge eines Azolderivats der folgenden Formel (I) aufgetragen wird:

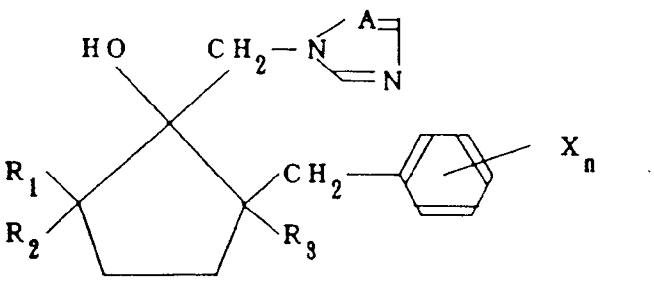

(I)

worin X ein Halogenatom oder eine $C_1$-$C_5$-Alkyl-, Halogenalkyl-, Phenyl-, Cyano- oder Nitrogruppe bedeutet, n für 0 oder eine ganze Zahl von 1 bis 5 steht, A ein Stickstoffatom oder CH darstellt, $R_1$ und $R_2$ individuell ein Wasserstoffatom oder eine $C_1$-$C_5$-Alkylgruppe bedeuten, $R_3$ ein Wasserstoffatom oder eine $C_1$-$C_3$-Alkylgruppe ist und, wenn n eine ganze Zahl von 2 bis 5 ist, die Reste X gleich oder unterschiedlich sein können.

2. Methode nach Anspruch 3, worin das industrielle Material ausgewählt ist unter Papier, Zellstoff, Fasern, Holz, Leder, Gummi, kunstharzen und Lacken.

## Revendications

1. Utilization d'un dérivé d'azole représenté par la formule suivante (I):

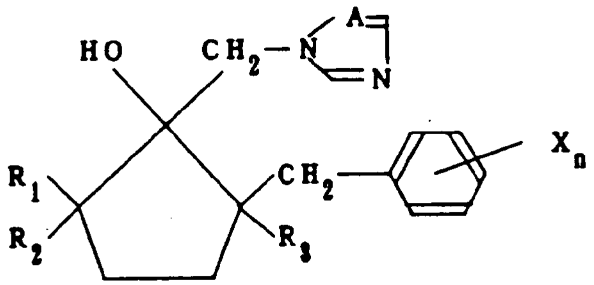

(I)

dans laquelle X rprésente un atome d'halogène ou un groupe alkyle en $C_{1-5}$ haloalkyle, phényle, cyano ou nitro, n est 0 ou un entier de 1-5, A représente un atome d'azote ou un radical CH, $R_1$ et $R_2$ représente chacun un atome d'hydrogène ou un groupe alkyle en $C_{1-5}$, $R_3$ représente un atome d'hydrogène ou un groupe alkyle en $C_{1-3}$, et lorsque n est un entier de 2-5, les X peuvent être identiques ou différents, en tant que biocide dans un matériau industriel.

2. Utilisation suivant la revendication 1, dans laquelle le matériau industriel est choisi parmi le papier, la pulpe, les fibres, le bois de charpente, le cuir, le caoutchouc, les résines de synthèse et les peintures.

3. Procédé pour empêcher la détérioration d'un matériau industriel, qui comprend l'application sur ce matériau industriel d'une quantité active en tant que biocide, d'un dérivé d'azole représenté par la formule suivante (I):

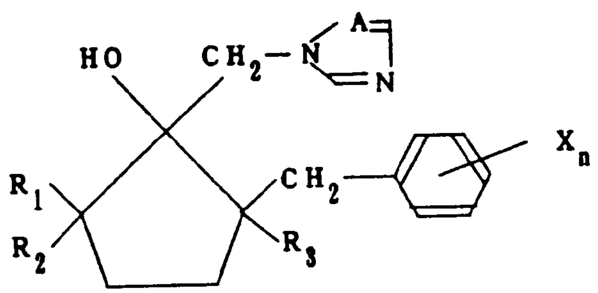

(I)

dans laquelle X représente un atome d'halogène ou un groupe alkyle en $C_{1-5}$, haloalkyle, phényle, cyano ou nitro, n est 0 ou un entier de 1-5, A représente un atome d'azote ou un radical CH, $R_1$ et $R_2$ représentent chacun un atome d'hydrogène ou un groupe alkyle en $C_{1-5}$, $R_3$ représente un atome d'hydrogène ou un groupe alkyle en $C_{1-3}$, et lorsque n est un entier de 2-5, les X peuvent être identiques ou différents.

4. Procédé suivant la revendication 3, dans lequel le matériau industriel est choisi parmi le papier, la pulpe, les fibres, le bois de charpente, le cuir, le caoutchouc, les résines de synthèse et les peintures.